# EUROPEAN PATENT APPLICATION

(11) **EP 4 640 248 A1**
(43) Date of publication of application: **29.10.2025**
(21) Application number: 23907820.7
(22) Date of filing: 21.12.2023
(51) Int. Cl.: A61L 27/18, A61L 27/50

(54) **DRY FORMULATION FOR TISSUE REPAIR**

(30) Priority: 23.12.2022 KR 20220183623
(71) Applicant: Samyang Holdings Corporation, Seoul 03129 (KR)
(72) Inventor: LEE, Jong Pil, Seoul 05668 (KR); PARK, Na Jeong, Yongin-si Gyeonggi-do 16852 (KR); PARK, Ji Hoon, Suwon-si Gyeonggi-do 16509 (KR); YOON, Hye Sung, Seongnam-si Gyeonggi-do 13544 (KR)
(74) Representative: Gleiss Große Schrell und Partner mbB
(86) International application number: PCT/KR2023/021287
(87) International publication number: WO 2024/136537

(57) **Abstract**

The present invention relates to a dry formulation for tissue repair and, specifically, to a dry formulation for tissue repair, comprising a copolymer of a hydrophilic biocompatible polymer and a hydrophobic biocompatible polymer, and an additive, wherein the formulation exhibits excellent tissue repair effects upon injection into the human body after reconstruction in an aqueous medium.

## Description

### TECHNICAL FIELD

The present invention relates to a dry formulation for tissue repair, and more specifically, a dry formulation for tissue repair which comprises a copolymer of hydrophilic biocompatible polymer and hydrophobic biocompatible polymer, and additives, and shows an excellent tissue repair effect after being reconstituted in an aqueous medium and introduced into the body.

### BACKGROUND ART

Recently, many people are interested in well-aging, which is growing old gracefully from a young age. It is no exaggeration to say that the current beauty market is focusing on anti-aging, which is aging slowly, beautifully and healthily. One of the most common signs of aging is a decrease in volume. In particular, since no volume in the face can make older and shabby look, people are very interested in fillers that can provide volume. Accordingly, the filler market is growing rapidly every year, and currently forms a market worth more than 2 trillion won worldwide.

Various substances are currently used as filler materials, and among them hyaluronic acid fillers occupy more than 90% of the global filler market, but have problems that the half-life in the body is within 1 to 3 days and so the persistence is very low and the resorption in the body occurs very rapidly. Thus, products having a resorption period extended by connecting hyaluronic acid with crosslinking material are on market. However, in case of such crosslinked products, because the crosslinking material, BDDE (1,4-butanediol diglycidyl ether), is a toxic carcinogen, there are problems that process cost increases according to removal process thereof, and loss is caused by product disposal due to microbial contamination, product disposal due to detection of residues, etc.

Accordingly, many tissue repair products using polymers that decompose in vivo have been developed, and as filler formulations using conventional biocompatible polymers, formulations have been developed by processing water-insoluble polymers into micro-sized particles and then dispersing them through a viscous excipient or thickener, and used. For example, a formulation in which poly(lactic acid) (PLA) particles with a diameter of 20 to 50 micrometers are dispersed in an aqueous solution of carboxymethylcellulose (CMC) or a formulation in which poly(caprolactone) (PCL) particles with a diameter of 20 to 50 micrometers are dispersed in an aqueous solution of CMC and glycerin have been used, but such formulations have problems of inconvenience in surgery due to needle clogging by the microparticles during injection and failure to obtain uniform tissue repair effect due to not uniformly dispersed particles.

Thus, there is an urgent need to develop a tissue repair product that can secure functionality, properties and safety suitable for tissue repair biomaterials without the risk of toxicity caused by a chemical crosslinking process, and a method for preparing the same.

### CONTENTS OF THE INVENTION

### PROBLEMS TO BE SOLVED

The purpose of the present invention is to provide a tissue repair product which, as a dry formulation, is easily transported, stored and handled, shows an excellent tissue repair effect after being reconstituted in an aqueous medium and introduced into the body, has no risk of toxicity, and can secure functionality, properties and safety suitable for tissue repair biomaterials without risk of toxicity.

### TECHNICAL MEANS

In an aspect, the present invention provides a dry formulation for tissue repair, comprising: a biocompatible copolymer, which is a copolymer of hydrophilic biocompatible polymer and hydrophobic biocompatible polymer; and additive; wherein, when the dry formulation is reconstituted in an aqueous medium, a composition reconstituted thereby exhibits thixotropic property of viscosity increase over time.

In other aspect, the present invention provides a method for preparing a dry formulation for tissue repair, the method comprising the steps of: (1) dissolving or dispersing a biocompatible copolymer, which is a copolymer of hydrophilic biocompatible polymer and hydrophobic biocompatible polymer, and additive in a mixture of an organic solvent and an aqueous medium; and (2) drying the product of said step (1); wherein, when the product of said step (2) is reconstituted in an aqueous medium, a composition reconstituted thereby exhibits thixotropic property of viscosity increase over time.

In another aspect, the present invention provides an injection composition for tissue repair, comprising the dry formulation for tissue repair; and a pharmaceutically acceptable carrier for injection.

In still another aspect, the present invention provides a method for preparing an injection composition for tissue repair, the method comprising: mixing the dry formulation for tissue repair and a pharmaceutically acceptable carrier for injection at room temperature.

### EFFECT OF THE INVENTION

The dry formulation for tissue repair of the present invention is easily transported, stored and handled, and when it is reconstituted in an aqueous medium, a composition reconstituted thereby exhibits thixotropic property of viscosity increase over time and thus shows an excellent tissue repair effect after being introduced into the body, and also there is no risk of toxicity, and functionality, properties and safety suitable for tissue repair biomaterials can be secured.

### BRIEF EXPLANATION OF THE DRAWINGS

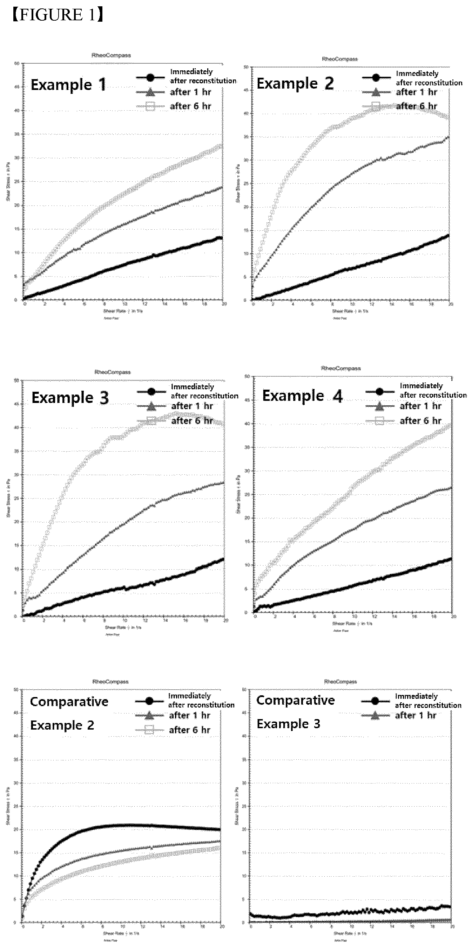

Figure 1 shows the results of the shear stress measurements conducted for the compositions of each of the dry formulations prepared in the present Examples 1 to 4 and Comparative Example 2 reconstituted in an aqueous medium, immediately after reconstitution and at a certain time after reconstitution (axis X: shear rate (1/s), axis Y: shear stress (Pa)). However, in case of Comparative Example 3, the results are those of the shear stress measurements conducted for the prepared dispersion formulation immediately after preparation and at 1 hour after preparation.
Figure 2 shows the results of animal tests conducted in Experimental Example 3 of the present invention.
Figure 3 shows the criteria for score evaluation conducted in Experimental Example 3 of the present invention.

### CONCRETE MODE FOR CARRYING OUT THE INVENTION

The present invention will be explained in detail below.

The dry formulation for tissue repair of the present invention comprises a biocompatible copolymer, which is a copolymer of hydrophilic biocompatible polymer and hydrophobic biocompatible polymer; and additive.

In the present invention, the dry formulation includes all formulations in a dried state, and there is no special limitation to its form, and also there is no special limitation to the drying method for its preparation, and for example, it can be dried by a method such as freeze drying, reduced pressure drying, other drying method (e.g. spin drying, spray drying), etc. into a form such as powder, etc., but it is not limited thereto.

In an embodiment, the dry formulation for tissue repair of the present invention may be in the form of powder, sponge, plug (rod shape) or bead, but it is not limited thereto.

In an embodiment, the hydrophilic biocompatible polymer may be selected from the group consisting of polyethylene glycol or its derivative (e.g., alkoxy- or hydroxy-polyethylene glycol), polyvinylpyrrolidone, polyvinyl alcohol, polyacrylamide, and a combination thereof, and more concretely, it may be selected from the group consisting of polyethylene glycol (PEG), methoxypolyethylene glycol (mPEG), and a combination thereof.

In an embodiment, the hydrophobic biocompatible polymer may be polymer of alpha (α)-hydroxy acid-derived monomers, and more concretely, it may be selected from the group consisting of polylactide, polyglycolide, poly(lactic-glycolide), polymandelic acid, polycaprolactone, polydioxan-2-one, polyamino acid, polyorthoester, polyanhydride, polycarbonate, polytrimethylenecarbonate, poly-β-hydroxybutyrate, polyhydroxyvalerate and a combination thereof, and still more concretely, it may be selected from the group consisting of polylactide, polyglycolide, poly(lactic-glycolide) and a combination thereof.

Specifically, the number average molecular weight (unit: g/mol) by GPC of the hydrophilic biocompatible polymer (Mn1) may be 1,000 or more, 2,000 or more, 3,000 or more, 4,000 or more, 5,000 or more, 6,000 or more, 7,000 or more, 8,000 or more, or 9,000 or more, and also it may be 30,000 or less, 29,000 or less, 28,000 or less, 27,000 or less, 26,000 or less, 25,000 or less, 24,000 or less, 23,000 or less, 22,000 or less, or 21,000 or less, but it is not limited thereto.

Specifically, the number average molecular weight (unit: g/mol) by GPC of the hydrophobic biocompatible polymer (Mn2) may be 500 or more, 1,000 or more, 2,000 or more, 3,000 or more, 4,000 or more, or 5,000 or more, and also it may be 20,000 or less, 19,000 or less, 18,000 or less, 17,000 or less, 16,000 or less, 15,000 or less, 14,000 or less, 13,000 or less, 12,000 or less, or 11,000 or less, but it is not limited thereto.

Specifically, the total number average molecular weight by GPC of the biocompatible copolymer may be 1,500 or more, 2,000 or more, 5,000 or more, 7,000 or more, 9,000 or more, 10,000 or more, 12,000 or more, or 14,000 or more, and also it may be 50,000 or less, 48,000 or less, 45,000 or less, 42,000 or less, 40,000 or less, 38,000 or less, 35,000 or less, or 32,000 or less, but it is not limited thereto.

In an embodiment, in the biocompatible copolymer, the ratio (Mn1/Mn2) of the number average molecular weight (Mn1) of the hydrophilic biocompatible polymer to the number average molecular weight (Mn2) of the hydrophobic biocompatible polymer may be 2.5 or less.

Specifically, the number average molecular weight ratio (Mn1/Mn2) of the hydrophilic biocompatible polymer to the hydrophobic biocompatible polymer may be 2.5 or less, 2.4 or less, 2.3 or less, or 2.2 or less, and also it may be 0.6 or more, 0.7 or more, 0.8 or more, or 0.9 or more, but it is not limited thereto.

The number average molecular weights of the hydrophilic and hydrophobic biocompatible polymers may be measured, for example, by gel permeation chromatography (GPC).

Gel permeation chromatography (GPC) is a method wherein the physical elution mechanism is that large components are eluted first and small components are eluted later depending on the size of the hydrodynamic volume of the analyzed components, and thus large molecules cannot enter the pores of the porous gel and pass through quickly while small molecules can enter the pores of the gel and be retained therein and thus pass through slowly, and the relative molecular weights are analyzed in the order of molecules passing through the column more quickly.

In an embodiment, the biocompatible copolymer may be prepared by a method comprising the steps of: polymerizing monomers for the above-explained hydrophobic biocompatible polymer in the presence of the above-explained hydrophilic biocompatible polymer to prepare a copolymer of hydrophilic biocompatible polymer and a hydrophobic biocompatible polymer; and drying the prepared copolymer, but it is not limited thereto.

The step of polymerizing monomers for the hydrophobic biocompatible polymer in the presence of the hydrophilic biocompatible polymer can be conducted according to known method and condition.

Specifically, the drying of the biocompatible copolymer can be conducted by freeze drying or other drying method (e.g., spin drying, etc.), and more concretely it can be conducted by freeze drying, but it is not limited thereto. The freeze drying of the biocompatible copolymer can be conducted in the presence of freeze-drying aid as explained above, but it is not limited thereto.

In an embodiment, the additive may be selected from the group consisting of buffer, freeze-drying aid, isotonic agent, or a combination thereof.

In an embodiment, the buffer may be selected from the group consisting of monobasic sodium phosphate (NaH₂PO₄), dibasic sodium phosphate (Na₂HPO₄), monobasic potassium phosphate (KH₂PO₄), dibasic potassium phosphate (K₂HPO₄), sodium citrate, sodium acetate, sodium bicarbonate, sodium carbonate, or a combination thereof.

The buffer includes its hydrates. That is, for example, the "dibasic sodium phosphate" includes hydrates (e.g., dihydrate, heptahydrate, etc.) of dibasic sodium phosphate.

In an embodiment, the buffer may comprise combination of dibasic sodium phosphate and monobasic sodium phosphate.

In an embodiment, in case where the buffer comprises combination of dibasic sodium phosphate (or dibasic potassium phosphate) and monobasic sodium phosphate (or monobasic potassium phosphate), the molar ratio of dibasic sodium phosphate (or dibasic potassium phosphate) : monobasic sodium phosphate (or monobasic potassium phosphate) may be 6~8 : 4~2, more concretely 6.5~7.5 : 3.5~2.5, and still more concretely 6.8~7.2 : 3.2~2.8, but it is not limited thereto.

In an embodiment, the phosphate buffer may be used in a suitable amount, for example, an amount of 5 mg to 10 mg per 1 g of the biocompatible copolymer, but it is not limited thereto.

In an embodiment, the freeze-drying aid (also called freeze-drying agent) may be one or more selected from the group consisting of lactose, maltose, sucrose, trehalose, mannitol, sorbitol, maltitol, xylitol, and lactitol.

In an embodiment, the freeze-drying aid may be used in an amount of 0.1 to 20 % by weight, more concretely 0.5 to 10 % by weight, but it is not limited thereto.

In an embodiment, the isotonic agent may be selected from the group consisting of sodium chloride, potassium chloride, calcium chloride, glucose, boric acid, or a combination thereof, but it is not limited thereto.

In an embodiment, the isotonic agent may be used in an amount of 0.1 to 20 % by weight, more concretely 0.5 to 10 % by weight, but it is not limited thereto.

In an embodiment, the dry formulation for tissue repair of the present invention may further comprise a local anesthetic.

Specifically, the local anesthetic may be one or more selected from the group consisting of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dicyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicyl alcohol, tetracaine, tolycaine, trimecaine, zolamine, and salts thereof.

In an embodiment, the dry formulation for tissue repair of the present invention may further comprise one or more components selected from the group consisting of polynucleotide fraction, polynucleotide fragment, biocompatible polymer, or a combination thereof.

Specifically, the polynucleotide fraction or fragment means a polymer consisting of nucleotide units, and preferably it may comprise nucleotide units including adenine (A), guanine (G), cytosine (C), thymine (T) and uracil (U). The units also may be modified, and the modified units may include 4-acetylcytidine, 5-(carboxyhydroxylmethyl)uridine, 2-O-methylcytidine, 5-carboxymethylaminomethyl-2-thiouridine, 5-carboxymethylamino-methyluridine, dihydrouridine, 2-O-methylpseudouridine, 2-O-methylguanosine, inosine, N6-isopentyladenosine, 1-methyladenosine, 1-methylpseudouridine, 1-methylguanosine, 1-methylinosine, 2,2-dimethylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, 5-methoxyuridine, 5-methoxycarbonylmethyl-2-thiouridine, 5-methoxycarbonylmethyluridine, 2-methylthio-N6-isopentyladenosine, uridine-5-oxyacetic acid-methyl ester, uridine-5-oxyacetic acid, wybutoxosine, wybutosine, pseudouridine, queuosine, 2-thiocitidine, 5-methyl-2-thiouridine, 2-thiouridine, 4-thiouridine, 5-methyluridine, 2-O-methyl-5-methyluridine, or 2-O-methyluridine, others, etc., but it is not limited thereto.

Also, the polynucleotides are naturally occurring nucleic acids, for example, deoxyribonucleic acid (DNA) and ribonucleic acid (RNA), and may be single-stranded or doublestranded. In addition, they may include nucleic acid analogues, and the nucleic acid analogues include non-naturally occurring bases, nucleotides that engage in linkage with nucleotides other than naturally occurring phosphodiester bonds, or nucleotides containing bases attached through linkages other than phosphodiester bonds. The nucleic acid analogues include phosphorothioate, phosphorodithioate, phosphorotriester, phosphoramidate, boranophosphate, methylphosphonate, chiral-methyl phosphonate, 2-O-methyl ribonucleotide, or peptide-nucleic acid (PNA), others, etc., but it is not limited thereto. The polynucleotide also may be a polynucleotide containing synthetic or modified nucleotide therein. Many different types of modifications to oligonucleotides are known in the art.

Specifically, the biocompatible polymer may be hyaluronic acid. The hyaluronic acid refers to a meaning including all of hyaluronic acid itself and salts and derivatives thereof, and may include all of aqueous solutions of hyaluronic acid, aqueous solutions of hyaluronic acid salt, and aqueous solutions of mixture thereof. The hyaluronic acid salt may be one or more selected from the group consisting of sodium hyaluronate, potassium hyaluronate, calcium hyaluronate, magnesium hyaluronate, zinc hyaluronate, cobalt hyaluronate, and tetrabutylammonium hyaluronate.

The dry formulation for tissue repair of the present invention may be prepared by dissolving or dispersing the above-explained biocompatible copolymer in a suitable solvent and drying it, but it is not limited thereto.

Specifically, the other aspect of the present invention provides a method for preparing a dry formulation for tissue repair, the method comprising the steps of: (1) dissolving or dispersing a biocompatible copolymer, which is a copolymer of hydrophilic biocompatible polymer and hydrophobic biocompatible polymer, and additive in a mixture of an organic solvent and an aqueous medium; and (2) drying the product of said step (1); wherein, when the product of said step (2) is reconstituted in an aqueous medium, a composition reconstituted thereby exhibits thixotropic property of viscosity increase over time.

According to an embodiment, in the method for preparing a dry formulation for tissue repair, the biocompatible copolymer, which is a copolymer of hydrophilic biocompatible polymer and hydrophobic biocompatible polymer, may be dissolved or dispersed in an aqueous medium comprising organic solvent and additive (for example, buffer), and then the resulting liquid may be freeze-dried.

In an embodiment, the organic solvent may be one or more selected from the group consisting of ketone-based solvent, alcohol-based solvent, sulfone-based solvent, nitrile-based solvent, ether-based solvent, amide-based solvent, alkane-based solvent and a combination thereof, and more concretely, it may be a ketone-based solvent or a mixture solvent comprising it, but it is not limited thereto.

In an embodiment, the ketone-based solvent may consist of, or comprise, one or more of substituted or unsubstituted linear or cyclic aliphatic ketone, more concretely linear or cyclic aliphatic ketone having a total carbon number of 3 to 10, still more concretely linear or cyclic aliphatic ketone having a total carbon number of 3 to 7, and still more concretely linear aliphatic ketone having a total carbon number of 3 to 5. For example, the ketone-based solvent may be acetone, methylethylketone, diethylketone, dipropylketone, cyclohexanone, 4-hydroxy-4-methyl-2-pentanone, methyl-n-propylketone, methyl-n-butylketone, methyl-i-butylketone, methyl-n-amylketone (or 2-heptanone) or a combination thereof, but it is not limited thereto.

In an embodiment, the alcohol-based solvent may consist of, or comprise, one or more of aliphatic alcohol having a total carbon number of 1 to 6, and more concretely aliphatic alcohol having a total carbon number of 1 to 4. For example, the alcohol-based solvent may be ethanol, methanol, t-butanol, isopropanol or a combination thereof, but it is not limited thereto.

In an embodiment, the sulfone-based solvent may consist of, or comprise, one or more of sulfoxide having a total carbon number of 2 to 6, and more concretely sulfoxide having a total carbon number of 2 to 4. For example, the sulfone-based solvent may be dimethyl sulfoxide, diethyl sulfoxide or a combination thereof, but it is not limited thereto.

In an embodiment, the nitrile-based solvent may consist of, or comprise, one or more of nitrile having a total carbon number of 2 to 8, and more concretely nitrile having a total carbon number of 2 to 6. For example, the nitrile-based solvent may be acetonitrile, but it is not limited thereto.

In an embodiment, the ether-based solvent may consist of, or comprise, one or more of linear or cyclic aliphatic ether having a total carbon number of 2 to 8, and more concretely linear or cyclic aliphatic ether having a total carbon number of 2 to 6. For example, the ether-based solvent may be 1,4-dioxane, but it is not limited thereto.

In an embodiment, the amide-based solvent may consist of, or comprise, one or more of amide having a total carbon number of 2 to 8, and more concretely amide having a total carbon number of 2 to 6. For example, the amide-based solvent may be dimethylacetoamide, but it is not limited thereto.

In an embodiment, the alkane-based solvent may consist of, or comprise, one or more of linear or cyclic alkane having a total carbon number of 4 to 10, and more concretely linear or cyclic alkane having a total carbon number of 4 to 8. For example, the alkane-based solvent may be cyclohexane, but it is not limited thereto.

There is no special limitation to the amount of the organic solvent used, and any amount that can dissolve all of the biocompatible copolymer is sufficient. In an embodiment, the amount of the organic solvent used may be 1 ml to 10 ml, more concretely 1 ml to 5 ml, per 1 g of the biocompatible copolymer, but it is not limited thereto.

In an embodiment, the aqueous medium may be distilled water, purified water, deionized water, ultrapure water, saline water, or a combination thereof, but it is not limited thereto.

In an embodiment, the aqueous medium comprising additive (for example, buffer) may be phosphate buffer solution, but it is not limited thereto.

In an embodiment, the aqueous medium may be used in an amount of 2 ml to 40 ml, more concretely 4 ml to 20 ml, per 1 g of the biocompatible copolymer, but it is not limited thereto.

In an embodiment, the concentration of the biocompatible copolymer in the biocompatible copolymer dispersion may be 0.1 to 50% by weight, more concretely 1 to 30% by weight, but it is not limited thereto.

According to an embodiment, prior to freeze drying, a freeze-drying aid may be added to the copolymer solution or dispersion prepared by using organic solvent and aqueous medium.

In an embodiment, the freeze drying may be conducted at a temperature of -45°C to -15°C, and more concretely, it may be conducted at a temperature of -40°C to -20°C.

The dry formulation for tissue repair of the present invention is easily dissolved in aqueous medium at room temperature to form polymer particles, and after being introduced into the body, the particles undergo self-assembly through hydrophobic aggregation of hydrophobic polymer under the influence of the environment in the body to form a large structure, which is not phagocytosed by macrophages and induces collagen, resulting in exhibition of excellent tissue repair effect.

In addition, when the dry formulation for tissue repair of the present invention is reconstituted in an aqueous medium, a composition reconstituted thereby exhibits thixotropic property of shear stress (i.e., viscosity) increase over time, and thus it can provide excellent tissue repair effect when introduced into the human body.

In an embodiment, when the dry formulation for tissue repair of the present invention is reconstituted in an aqueous medium, a composition reconstituted thereby may exhibit that the shear stress measured at 1 hour after reconstitution is 1.5 times or greater than the shear stress measured immediately after reconstitution under the same condition.

More concretely, a composition of reconstituting the dry formulation for tissue repair of the present invention in an aqueous medium (for example, 25°C) to a concentration of 12.5 % by weight (hereinafter, 12.5 wt% reconstituted composition) may exhibit that the shear stress measured under a shear rate of 10/s at 1 hour after reconstitution (hereinafter, "shear stress (1 hour)") is 1.5 times or greater than the shear stress measured immediately after reconstitution under the same condition (hereinafter, "shear stress (initial)").

Still more concretely, the shear stress (1 hour) of the 12.5 wt% reconstituted composition may be 1.5 times or greater, 1.6 times or greater, 1.7 times or greater, 1.8 times or greater, 1.9 times or greater, 2 times or greater, 2.1 times or greater, 2.2 times or greater, 2.3 times or greater, 2.4 times or greater, 2.5 times or greater, 2.6 times or greater, 2.7 times or greater, 2.8 times or greater, 2.9 times or greater, or 3 times or greater than the shear stress (initial), but it is not limited thereto. In addition, the shear stress (1 hour) of the 12.5 wt% reconstituted composition may be 6 times or less, 5.5 times or less, 5 times or less, 4.5 times or less, or 4 times or less, but it is not limited thereto.

Also, in an embodiment, when the dry formulation for tissue repair of the present invention is reconstituted in an aqueous medium, a composition reconstituted thereby may exhibit that the shear stress measured at 6 hours after reconstitution is 2 times or greater than the shear stress measured immediately after reconstitution under the same condition.

More concretely, the 12.5 wt% reconstituted composition of the dry formulation for tissue repair of the present invention may exhibit that the shear stress measured under a shear rate of 10/s at 6 hours after reconstitution (hereinafter, "shear stress (6 hours)") is 2 times or greater than the shear stress (initial).

Still more concretely, the shear stress (6 hours) of the 12.5 wt% reconstituted composition may be
2 times or greater, 2.1 times or greater, 2.2 times or greater, 2.3 times or greater, 2.4 times or greater, 2.5 times or greater, 2.6 times or greater, 2.7 times or greater, 2.8 times or greater, 2.9 times or greater, 3 times or greater, 3.1 times or greater, 3.2 times or greater, 3.3 times or greater, 3.4 times or greater, 3.5 times or greater, 3.6 times or greater, 3.7 times or greater, 3.8 times or greater, 3.9 times or greater, or 4 times or greater than the shear stress (initial), but it is not limited thereto. In addition, the shear stress (6 hours) of the 12.5 wt% reconstituted composition may be 8 times or less, 7.5 times or less, 7 times or less, 6.5 times or less, 6 times or less, 5.5 times or less, or 5 times or less, but it is not limited thereto.

In the present invention, the tissue repair effect refers to the effect of returning the tissue to its original state when necrosis, defects, etc. occur in skin tissue due to trauma, inflammation, aging, etc.

Therefore, another aspect of the present invention provides an injection composition for tissue repair, comprising the dry formulation for tissue repair of the present invention; and a pharmaceutically acceptable carrier for injection.

As the pharmaceutically acceptable carrier for injection comprised in the injection composition for tissue repair of the present invention, conventional one can be used without limitation, and for example, it may be selected from the group consisting of distilled water for injection, physiological saline, 5% glucose, buffer solutions (e.g., phosphate buffer solution (PBS)), hyaluronic acid solution and a combination thereof, but it is not limited thereto.

In addition to the components explained above, the injection composition for tissue repair of the present invention may further comprise one or more conventional additives that can be used in injection formulation.

Still another aspect of the present invention provides a method for preparing an injection composition for tissue repair, the method comprising: mixing (preferably, mixing at room temperature) the dry formulation for tissue repair of the present invention and a pharmaceutically acceptable carrier for injection. In this, "room temperature" means 1 to 30°C, 20 to 30°C, 22 to 28°C, more concretely 24 to 26°C (e.g., 25°C).

The existing polymer product for tissue repair must be heated (e.g., the temperature must be raised between the melting point of the polymer and the boiling point of water) to be manufactured in the form of an aqueous solution. However, the dry formulation for tissue repair prepared by the method of the present invention is easily dissolved in aqueous medium even at room temperature, and thus its injectable composition in the form of an aqueous solution can be easily prepared and used at room temperature.

The present invention will be explained below in more detail through the following Examples and Comparative Examples. However, the scope of the present invention is not limited thereby in any manner.

### [EXAMPLES]

### Example 1

In order to obtain a biocompatible copolymer with a target number average molecular weight (Mn) of 15,000 g/mol, in the presence of methoxypolyethyleneglycol (mPEG) with a number average molecular weight (Mn1) of 9,850 g/mol (measured by GPC) as a hydrophilic biocompatible polymer, D,L-lactide monomer was polymerized in the presence of a catalyst, and then the resulting copolymer was dried to obtain a copolymer. As a result of GPC measurement, the number average molecular weight (Mn) of the prepared mPEG-Poly(D,L-lactide) copolymer was 15,500 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 1.74.

A dispersion solution of the copolymer was prepared by using a linear aliphatic ketone solvent having a total carbon number of 4 and a phosphate buffer. To the prepared dispersion solution, D-mannitol as a freeze-drying aid was added in an amount of 10 wt% to prepare a mixture for freeze-drying, and freeze-drying thereof was conducted to prepare a dry formulation for tissue repair in powder form.

### Example 2

Excepting that the target number average molecular weight (Mn) of the biocompatible copolymer was set to 17,000 g/mol, a powder formulation was prepared in the same manner as Example 1. As a result of GPC measurement, the number average molecular weight (Mn) of the prepared mPEG-Poly(D,L-lactide) copolymer was 17,100 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 1.36.

By using the prepared copolymer, a dry formulation for tissue repair in powder form was prepared in the same manner as Example 1.

### Example 3

Excepting that the target number average molecular weight (Mn) of the biocompatible copolymer was set to 20,000 g/mol, a powder formulation was prepared in the same manner as Example 1. As a result of GPC measurement, the number average molecular weight (Mn) of the prepared mPEG-Poly(D,L-lactide) copolymer was 20,200 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 0.95.

By using the prepared copolymer, a dry formulation for tissue repair in powder form was prepared in the same manner as Example 1.

### Example 4

Excepting that a linear aliphatic ketone solvent having a total carbon number of 3 was used, a dry formulation for tissue repair in powder form was prepared in the same manner as Example 1 by using the biocompatible copolymer prepared in Example 1.

### Comparative Example 1

Miracle L, a liquid-type commercial product known to generate collagen, was used as Comparative Example 1.

### Comparative Example 2

Excepting that phosphate buffer only was used without organic solvent in preparing polymer dispersion solution, a dry formulation for tissue repair in powder form was prepared in the same manner as Example 1 by using the biocompatible copolymer prepared in Example 1.

### Comparative Example 3

Excepting that phosphate buffer only was used without organic solvent, a dispersion solution was prepared in the same manner as Example 1 by using the biocompatible copolymer prepared in Example 1. To the prepared dispersion solution, D-mannitol as a freeze-drying aid was added in an amount of 10 wt%. The concentration of the dispersion solution was the same as the concentration reconstituted in Experimental Example 2.

### Experimental Example 1: Analysis of polymer molecular weight

The number average molecular weights (Mn) of the copolymers prepared in Examples 1 to 3 were measured by performing Gel Permeation Chromatography (GPC) under the conditions shown in the following table.

| | |
|---|---|
| Instrument | Agilent Infinity 1260 GPC/SEC system |
| Column | One Mixed-C guard column and two Mixed-C analytical columns |
| Eluent | Chloroform (HPLC grade) |
| Detector | Refractive Index (RI) Detector |
| Flow rate | 1.0 mL/min |
| Temperature | 35°C |
| Concentration | 0.1~ 0.2 w/v % |
| Injection volume | 100 µL |
| Standard material | 9 kinds of PS (195300/69650/30230/19500/12980/6320/3090/1290/945g/mol) |

### Experimental Example 2: Measurement of shear stress

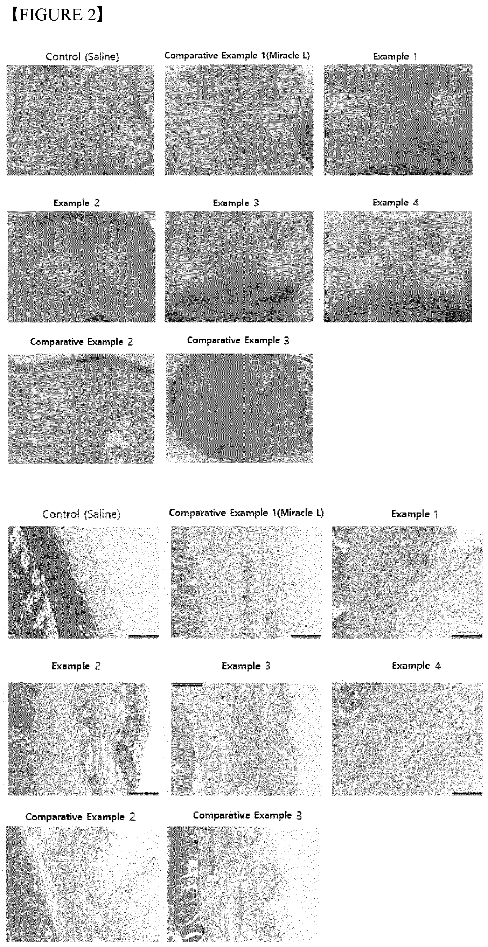

Each of the dry formulations for tissue repair of Examples 1 to 4 and Comparative Example 2 was reconstituted as an aqueous composition with 12.5 wt% concentration by adding water for injection thereto, and the shear stress of the reconstituted composition was measured immediately after reconstitution, and at 1 hour and 6 hours after reconstitution, by using Rheometer while increasing the shear rate from 0.1/s to 10/s. In case of Comparative Example 3, the shear stress was measured immediately after preparation and at 1 hour after preparation. The results are shown in the following Table 1 and Figure 2.

### Rheometer (MCR102e, Anton Paar) parameter setting

Temperature: 25°C
Shear rate: 0.1~10/s (ramp linear)
Loading volume: 500 µl
Plate diameter: 25 mm
Gap between plate and glass: 0.8 mm
Result: Shear rate versus Shear stress

**[Table 1] Shear stress measured at shear rate of 10/s**

| | **Shear stress (Pa, immediately after reconstitution^{*})** | **Shear stress (Pa, at 1 hour after reconstitution^{*})** | **Shear stress (Pa, at 6 hours after reconstitution)** |
|---|---|---|---|
| **Example 1** | 13.1 | 23.8 | 32.6 |
| **Example 2** | 12.2 | 28.5 | 40.6 |
| **Example 3** | 13.9 | 34.8 | 39.0 |
| **Example 4** | 11.2 | 26.5 | 39.7 |
| **Comparative Example 2** | 20.0 | 17.4 | 16.0 |
| **Comparative Example 3** | 2.8 | 2.4 | N.A. |

| | | | |
|---|---|---|---|
| *: meaning "preparation of dispersion solution" in Comparative Example 3 | | | |

### Experimental Example 3: Verification of efficacy as a formulation for tissue repair through animal tests

The efficacy for tissue repair of the dry formulation in powder form according to the present invention was verified through animal tests. The animal tests were conducted using 5-week-old SD rats (purchased from Orient Bio Co., Ltd.).

In the animal tests, saline and test materials were administered to each 5-week-old SD rat on both sides. During the test period, the rearing environment was set at a temperature of 24 ± 2°C, relative humidity of 50 ± 10%, and lighting time of 12 hours, and food was allowed to be eaten freely.

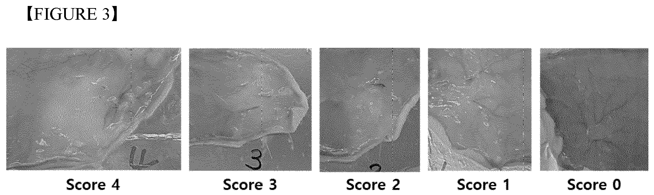

Physiological saline as Control, a reconstituted composition of each dry formulation in powder form prepared in Examples 1 to 4 and Comparative Example 2 dissolved in water for injection (12.5 wt% concentration) as a test material, and liquid formulations of Comparative Example 1 (Miracle L, a liquid-type commercial product) and Comparative Example 3 (dispersion solution) were injected in an amount of 100 µl constantly, and after 4 weeks, the test animals were sacrificed, and the skin tissue at the sample injection site (the area indicated by the arrow in the upper part of Figure 3) was stained with Masson's Trichrome (MT) and collagen formation in the tissue was to observed in order to evaluate the ability of new collagen biosynthesis. The results are shown in Figure 3 (the lower part of Figure 3).

In addition, for the sites where the new collagen was generated, score evaluation was conducted according to the following criteria, and the results are shown in the following Table 2.

| **Grade** | **Evaluation criteria (with reference to** **Figure 3****)** |
|---|---|
| **Score 4** | Product having clear boundaries with a sense of volume |
| **Score 3** | Product having clear boundaries without a sense of volume |
| **Score 2** | Product having unclear boundaries observed without visible capillaries under the product |
| **Score 1** | Product having unclear boundaries observed with visible capillaries under the product |
| **Score 0** | No product observed as compared with Control |

**[Table 2]**

| | Control (saline) | Comparative Example 1 (Miracle L) | Example 1 | Example 2 | Example 3 | Example 4 | Comparative Example 2 | Comparative Example 3 |
|---|---|---|---|---|---|---|---|---|
| Score 0 | 100% | 0 | 0 | 0 | 0 | 0 | 50% | 50% |
| Score 1 | 0% | 0 | 0 | 0 | 5% | 0 | 45% | 50% |
| Score 2 | 0 | 10% | 0 | 0 | 5% | 0 | 5% | 0 |
| Score 3 | 0 | 35% | 20% | 15% | 5% | 5% | 0 | 0 |
| Score 4 | 0 | 55% | 80% | 85% | 85% | 95% | 0 | 0 |

In addition, by measuring the thicknesses of the sites where the new collagen was generated, the relative evaluation was conducted as compared with the thickness of Control, and the results are shown in the following Table 3.

**[Table 3]**

| | Control (saline) | Comparative Example 1 (Miracle L) | Example 1 | Example 2 | Example 3 | Example 4 | Comparative Example 2 | Comparative Example 3 |
|---|---|---|---|---|---|---|---|---|
| Relative thickness | 1 | 3.4 | 5.8 | 5.1 | 4.8 | 6.1 | 3.0 | 2.6 |

From the results of Figure 3 and Tables 2 and 3, it was confirmed that, as compared with Comparative Examples, the reconstituted compositions of the dry formulations of Examples showed excellently better collagen formation, and in particular, formulation of a large number of collagen fibers which were significantly thicker than Comparative Example 1, Miracle L.

That is, it was confirmed thereby that, as compared with the commercial product of Comparative Example 1 (Miracle L), the dry formulation according to the present invention has advantages that it is easily transported, stored and handled, and easily dissolved in an aqueous medium at room temperature, and when introduced into the body, it induces collagen formation more excellently, resulting in exhibition of more excellent tissue repair effect.

## Claims

1. A dry formulation for tissue repair, comprising:
a biocompatible copolymer, which is a copolymer of hydrophilic biocompatible polymer and hydrophobic biocompatible polymer; and additive;
wherein, when the dry formulation is reconstituted in an aqueous medium, a composition reconstituted thereby exhibits thixotropic property of viscosity increase over time.

2. The dry formulation for tissue repair of Claim 1, wherein the hydrophilic biocompatible polymer is selected from the group consisting of polyethylene glycol or its derivative, polyvinylpyrrolidone, polyvinyl alcohol, polyacrylamide and a combination thereof.

3. The dry formulation for tissue repair of Claim 1, wherein the hydrophobic biocompatible polymer is selected from the group consisting of polylactide, polyglycolide, poly(lactic-glycolide), polycaprolactone, polydioxan-2-one, polyamino acid, polyorthoester, polyanhydride, polycarbonate and a combination thereof,

4. The dry formulation for tissue repair of Claim 1, wherein the hydrophilic biocompatible polymer is selected from the group consisting of polyethylene glycol (PEG), methoxypolyethylene glycol (mPEG) and a combination thereof, and the hydrophobic biocompatible polymer is selected from the group consisting of polylactide, polyglycolide, poly(lactic-glycolide) and a combination thereof.

5. The dry formulation for tissue repair of Claim 1, wherein the additive is selected from the group consisting of buffer, freeze-drying aid, isotonic agent, or a combination thereof.

6. The dry formulation for tissue repair of Claim 5, wherein the buffer is selected from the group consisting of monobasic sodium phosphate (NaH₂PO₄), dibasic sodium phosphate (Na₂HPO₄), monobasic potassium phosphate (KH₂PO₄), dibasic potassium phosphate (K₂HPO₄), sodium citrate, sodium acetate, sodium bicarbonate, sodium carbonate, or a combination thereof.

7. The dry formulation for tissue repair of Claim 5, wherein the freeze-drying aid is one or more selected from the group consisting of lactose, maltose, sucrose, trehalose, mannitol, sorbitol, maltitol, xylitol and lactitol.

8. The dry formulation for tissue repair of Claim 5, wherein the isotonic agent is selected from the group consisting of sodium chloride, potassium chloride, calcium chloride, glucose, boric acid, or a combination thereof.

9. The dry formulation for tissue repair of Claim 1, wherein, when the dry formulation is reconstituted in an aqueous medium, a composition reconstituted thereby exhibits that the shear stress measured at 1 hour after reconstitution is 1.5 times or greater than the shear stress measured immediately after reconstitution under the same condition.

10. The dry formulation for tissue repair of Claim 1, wherein, when the dry formulation is reconstituted in an aqueous medium, a composition reconstituted thereby exhibits that the shear stress measured at 6 hours after reconstitution is 2 times or greater than the shear stress measured immediately after reconstitution under the same condition.

11. A method for preparing a dry formulation for tissue repair, the method comprising the steps of:
(1) dissolving or dispersing a biocompatible copolymer, which is a copolymer of hydrophilic biocompatible polymer and hydrophobic biocompatible polymer, and additive in a mixture of an organic solvent and an aqueous medium; and
(2) drying the product of said step (1);
wherein, when the product of said step (2) is reconstituted in an aqueous medium, a composition reconstituted thereby exhibits thixotropic property of viscosity increase over time.

12. The method for preparing a dry formulation for tissue repair of Claim 11, wherein the organic solvent is one or more selected from the group consisting of ketone-based solvent, alcohol-based solvent, sulfone-based solvent, nitrile-based solvent, ether-based solvent, amide-based solvent, alkane-based solvent, and a combination thereof.

13. An injection composition for tissue repair, comprising:
the dry formulation for tissue repair of any one of Claims 1 to 10; and
a pharmaceutically acceptable carrier for injection.

14. The injection composition for tissue repair of Claim 13, wherein the dry formulation for tissue repair or the carrier for injection comprises a local anesthetic.

15. The injection composition for tissue repair of Claim 13, further comprising one or more components selected from the group consisting of polynucleotide fractions, polynucleotide fragments, biocompatible polymers, or a combination thereof.

16. A method for preparing an injection composition for tissue repair, the method comprising: mixing the dry formulation for tissue repair of any one of Claims 1 to 10 and a pharmaceutically acceptable carrier for injection.
